# EUROPEAN PATENT APPLICATION

(11) **EP 4 656 073 A1**
(43) Date of publication of application: **03.12.2025**
(21) Application number: 24187044.3
(22) Date of filing: 08.07.2024
(51) Int. Cl.: A24F 40/40

(54) **AEROSOL DELIVERY SYSTEMS AND METHODS**

(30) Priority: 27.05.2024 CN 202410667690
(71) Applicant: Nicoventures Trading Limited, London WC2R 3LA (GB)
(72) Inventor: LI, Ruifan, London WC2R 3LA (GB); LI, Bingquan, London WC2R 3LA (GB); FAN, Zhimin, London WC2R 3LA (GB); LI, Kwong Wing, London WC2R 3LA (GB); NANDRA, Charanjit, London WC2R 3LA (GB); KIM, Edo, London WC2R 3LA (GB); HUI, King Pun, London WC2R 3LA (GB); XELHUANTZI, Jorge Luis Gomez, London WC2R 3LA (GB)
(74) Representative: D Young & Co LLP

(57) **Abstract**

An aerosol delivery system comprising: a power supply; and a retention mechanism for securing the power supply within the system, wherein the retention mechanism comprises: a first component and a second component removably engagable with the first component, wherein the retention mechanism is configured to plastically deform or fracture during disengagement.

## Description

### Field

This disclosure relates to aerosol delivery systems, which may include nicotine delivery systems.

### Background

Aerosol delivery systems such as electronic cigarettes (e-cigarettes) generally contain an aerosol generating material, such as a chamber of a source solid or liquid, which may contain an active substance and / or a flavour, from which an aerosol or vapour is generated for inhalation by a user, e.g. through heat vaporisation. An aerosol delivery system typically comprises an aerosol generation area containing an aerosol generator, e.g. a heating element, arranged to vaporise or aerosolise a portion of precursor material to generate a vapour or aerosol in the aerosol generation area. As a user inhales on the system and electrical power is supplied to the vaporiser, air is drawn into the system through an inlet hole and along an inlet air channel connecting to the aerosol generation area, where the air mixes with vaporised precursor material to form a condensation aerosol. There is an outlet channel connecting the aerosol generation area to an outlet in a mouthpiece and the air drawn into the aerosol generation area as a user inhales on the mouthpiece continues along the outlet flow path to the mouthpiece outlet, carrying the aerosol with it, for inhalation by the user.

Some electronic cigarettes may include a flavour element in the air flow path to impart additional flavours. Such systems may be referred to as hybrid devices, and the flavour element may, for example, include a portion of tobacco arranged in the air flow path between the aerosol generation area and the mouthpiece such that vapour / aerosol drawn through the device passes through the portion of tobacco before exiting the mouthpiece for user inhalation.

It is of interest to reduce the environmental impact of such systems.

### Brief summary of the invention

In one aspect, there is provided an aerosol delivery system comprising: a power supply; and a retention mechanism for securing the power supply within the system, wherein the retention mechanism comprises: a first component and a second component removably engagable with the first component, wherein the retention mechanism is configured to plastically deform or fracture during disengagement.

In a further aspect, there is provided a method of securing a power supply within an aerosol delivery system, comprising: providing a power supply and a retention mechanism comprising a first component and a second component removably engagable with the first component, the method comprising: locating the power supply within the system; and engaging the first component with the second component to secure the power supply within the system, wherein the retention mechanism is configured to plastically deform or fracture during disengagement.

The claimed invention further provides corresponding functional means and further provides additional embodiments as claimed in the dependent claims.

In some examples, the first component comprises a retention element. In some examples, the second component comprises a body having a connection portion. The retention element may be configured to engage the connection portion of the body.

In some examples, the first component is an external component. In some examples, the second component is an internal component, located internally within the system.

In some examples, the retention mechanism is configured to provide a snap-fit engagement. In other words, the first component may be removably engagable with the second component by snap-fit.

In some examples, the first component and/or the second component is/are configured to plastically deform or fracture during disengagement.

In some examples, the retention mechanism changes irreversibly upon disengagement of the first and second components. In some examples, the retention mechanism is configured to plastically deform or fracture during disengagement to prevent re-engagement of the first and second components after disengagement.

In some examples, the retention mechanism is a single-use retention mechanism.

In some examples, the system comprises a stress concentration mechanism configured to promote plastic deformation or fracture during disengagement.

In some examples, the retention mechanism comprises a weakened region configured to promote the plastic deformation or fracture during disengagement.

In some examples, the weakened region comprises a thinner region, a hollow region, a holey region and/or an undercut.

In some examples, the first component and/or the second component comprises a weakened region, such as a thinner region, a hollow region, a holey region and/or an undercut.

In some examples, the first or second component comprises one or more protrusions, configured to engage one or more complementary recesses or protrusions on the other of the first and second components.

In some examples, the first or second component comprising the one or more protrusions comprises the weakened region. In some examples, the first or second component comprises an opposing pair of `L'-shaped protrusions.

In some examples, the first or second component comprises a thinner region of, proximal to or surrounding the one or more protrusions, providing a stress concentration on, proximal to or surrounding the one or more protrusions.

In some examples, the first or second component comprises a cavity for receiving the power supply.

In some examples, the system, the first or the second component comprises a housing enclosing the power supply within the cavity.

In some examples, the first component comprises:
a) one or more protrusions or recesses at or proximal to a first end of the first component, the one or more protrusions or recesses configured to engage with the second component; and
b) a stepped portion at or proximal to a second, opposing end of the first component.

In some examples, the first component comprises an aperture for receiving a user's fingertip or fingernail.

In some examples, the stepped portion spaces the second, opposing end of the first component from a housing enclosing the power supply within the cavity.

In some examples, the first component comprises a pull-tab or ring-pull.

In some examples, when the first and second components are engaged, the retention mechanism is configured to plastically deform or fracture upon a linear movement of the first component relative to the second component. In other examples, when the first and second components are engaged, the retention mechanism is configured not to plastically deform or fracture upon a linear movement of the first component relative to the second component.

In some examples, when the first and second components are engaged, the retention mechanism is configured to plastically deform or fracture upon a rotational movement of the first component relative to the second component. In other examples, when the first and second components are engaged, the retention mechanism is configured not to plastically deform or fracture upon a rotational movement of the first component relative to the second component.

In some examples:
a. the first or second component comprises one or more protrusions configured to engage the other of the first and second components;
b. the first or second component comprises a cavity for receiving the power supply; and
c. the first or second component comprises a weakened region configured to promote plastic deformation or fracture of the retention mechanism during disengagement of the retention mechanism, preventing subsequent re-engagement of the retention mechanism.

In some examples:
a. the first or second component comprises one or more protrusions configured to engage complementary recesses or protrusions on the other of the first and second components;
b. the second component comprises a cavity for receiving the power supply; and
c. the first or second component comprises a weakened region configured to promote plastic deformation or fracture of the retention mechanism during disengagement of the retention mechanism, preventing subsequent re-engagement of the retention mechanism.

In some examples:
a. the system comprises a housing enclosing the power supply within the cavity; and
b. the housing comprises an aperture, wherein when the retention mechanism is engaged, the one or more protrusions of the first or second component pass through the aperture in the housing to engage the other of the first and second components.

In some examples, the system further comprises an aerosol generator.

In some examples, the aerosol delivery system comprises a controller. In some examples, the controller is configured to detect disengagement of the retention mechanism and, in response, disable the supply of power from the power supply to the system.

In some examples, the aerosol delivery system comprises aerosol-generating material, which may be a solid, liquid or gel, optionally stored in a chamber or a reservoir.

In some examples, the aerosol delivery system comprises a removable cartridge comprising aerosol-generating material.

In some examples, the method comprises locating the power supply within a cavity in the system and engaging the first component with the second component to secure the power supply within the cavity.

Also disclosed is a method of releasing a power supply secured within an aerosol delivery system by a retention mechanism comprising a first component and a second component removably engagable with the first component, the method comprising:
disengaging the retention mechanism, plastically deforming or fracturing the retention mechanism during disengagement; and
removing the power supply.

In some examples, the method comprises disengaging the retention mechanism by moving the first component relative to the second component. In some examples, the movement comprises linear or rotational movement of the first component relative to the second component.

### Brief description of the figures

Embodiments of the disclosure will now be described, by way of example only, with reference to the accompanying drawings, in which:
Figure 1 is a schematic cross-section view of an aerosol delivery system comprising a retention mechanism in accordance with some embodiments of the disclosure;
Figure 2 is a schematic cross-section view of another aerosol delivery system comprising a retention mechanism in accordance with some embodiments of the disclosure;
Figures 3-5 are top-down, side perspective and bottom-up perspective views respectively of a first retention mechanism component in accordance with some embodiments of the disclosure;
Figure 6 is a perspective view of a second retention mechanism component in accordance with some embodiments of the disclosure;
Figure 7 is a perspective view of a housing in accordance with some embodiments of the disclosure;
and
Figure 8 is a perspective view of a first retention mechanism component engaging the housing of figure 7, in accordance with some embodiments of the disclosure.

### Detailed description of the disclosure

Aspects and features of certain examples and embodiments are described herein. Some aspects and features may be implemented conventionally and these are not described in detail, for brevity.

The claimed invention may generally provide a sub-assembly or sub-system suitable for use in an aerosol delivery system, or configured for use in an aerosol delivery system. The sub-system may generally form part of an aerosol delivery system and in particular may form part of the reusable device part of a two-part system.

In particular, the claimed invention provides a retention mechanism for an aerosol delivery system that prevents direct access to a power source for the system during normal use, but permits access to the power source when the power source (or wider system) reaches end of life, so that the power source (which may be hazardous) can be separated from the system and recycled or otherwise disposed of safely and in an environmentally-friendly manner.

### Introduction

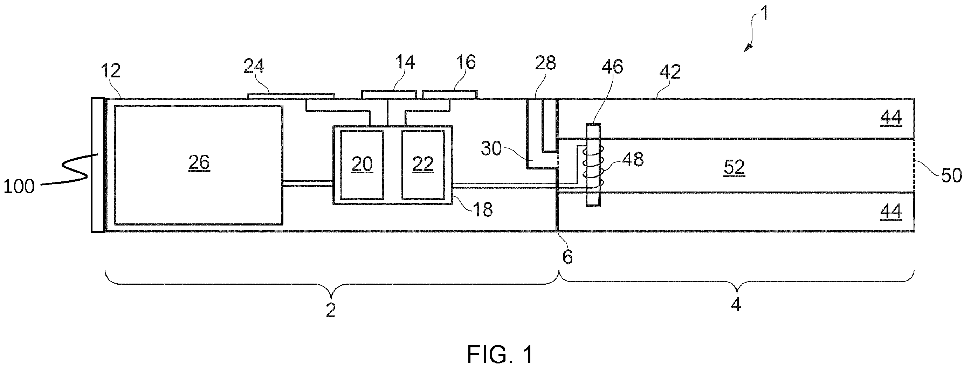

Figure 1 is a cross-sectional view through an example aerosol delivery system 1 in accordance with certain embodiments of the disclosure, providing an introduction to two-part aerosol delivery systems, the components therein and their functionality.

The aerosol delivery system 1 comprises two main parts, a reusable part 2 and a replaceable / disposable consumable cartridge part 4. In normal use, the reusable part 2 and the cartridge part 4 are releasably coupled together at an interface 6. When the cartridge part 4 is exhausted or the user wishes to switch to a different cartridge part 4, the cartridge part 4 may be removed from the reusable part 2 and a replacement cartridge part 4 attached to the reusable part 2 in its place. The interface 6 may provide a structural, electrical and airflow path connection between the two parts 2, 4 and may be established in accordance with conventional techniques, e.g. based around a screw thread, magnetic or bayonet fixing with electrical contacts and openings for the electrical connection and airflow path between the two parts 2, 4 as appropriate. The specific manner by which the cartridge part 4 mounts to the reusable part 2 is not significant to the principles described herein, but for the sake of a concrete example is assumed here to comprise a magnetic coupling (not represented in figure 1). It will also be appreciated the interface 6 in some implementations may not support an electrical and / or airflow path connection between the respective parts 2, 4. For example, in some implementations an aerosol generator may be provided in the reusable part 2 rather than in the cartridge part 4, or the transfer of electrical power from the reusable part 2 to the cartridge part 4 may be wireless (e.g. based on electromagnetic induction), so that an electrical connection between the reusable part 2 and the cartridge part 4 is not needed. Furthermore, in some implementations the airflow through the electronic cigarette might not go through the reusable part 2, so that an airflow path connection between the reusable part 2 and the cartridge part 4 is not needed. In some instances, a portion of the airflow path may be defined at the interface between portions of the reusable part 2 and cartridge part 4 when these are coupled together for use.

The cartridge / consumable part 4 may, in certain embodiments, be broadly conventional. In figure 1, the cartridge part 4 comprises a cartridge housing 42 formed of a plastics material. The cartridge housing 42 supports other components of the cartridge part 4 and provides the mechanical interface 6 with the reusable part 2. The cartridge housing 42 is generally circularly symmetrical about a longitudinal axis along which the cartridge part 4 couples to the reusable part 2. In this example, the cartridge part 4 has a length of around 4 cm and a diameter of around 1.5 cm. However, the specific dimensions, geometry, overall shapes and materials used may vary.

Within the cartridge housing 42 is a chamber or reservoir 44 that contains aerosol-generating material. In the example of figure 1, the reservoir 44 stores a supply of liquid aerosol generating material and the liquid reservoir 44 has an annular shape with an outer wall defined by the cartridge housing 42 and an inner wall that defines an airflow path 52 through the cartridge part 4. The reservoir 44 is closed at each end with end walls to contain the aerosol generating material. The reservoir 44 may be formed conventionally, e.g. comprising a plastics material and/or integrally moulded with the cartridge housing 42.

The cartridge / consumable part 4 further comprises an aerosol generator 48 located towards an end of the reservoir 44, opposite to a mouthpiece outlet 50. In a two-part system such as in figure 1, the aerosol generator 48 may be in either of the reusable part 2 or the cartridge part 4. For example, in some embodiments, the aerosol generator 48 (e.g. a heater, which may be in the form of a wick and coil arrangement as shown, a distiller, which may be formed from a sintered metal fibre material or other porous conducting material, or any suitable alternative aerosol generator) may be comprised in the reusable part 2, and is brought into proximity with a portion of aerosol generating material in the cartridge part 4 when the cartridge part 4 is engaged with the reusable part 2. In such embodiments, the cartridge part 4 may comprise a portion of aerosol generating material, and an aerosol generator 48 is at least partially inserted into or at least partially surrounds the portion of aerosol generating material as the cartridge part 4 is engaged with the reusable part 2.

In the example of figure 1, a wick 46 in contact with the aerosol generator 48 extends transversely across the cartridge airflow path 52 with its ends extending into the reservoir 44 of the liquid aerosol generating material through openings in the inner wall. The openings in the inner wall of the reservoir 44 are sized to broadly match the dimensions of the wick 46 to provide a reasonable seal against leakage from the reservoir 44 into the cartridge airflow path, without unduly compressing the wick 46, which may be detrimental to its fluid transfer performance.

The wick 46 and aerosol generator 48 are arranged in the cartridge airflow path 52 such that a region of the cartridge airflow path 52 around the wick 46 and heater 48 in effect defines a vaporisation region for the cartridge part 4. Aerosol generating material in the reservoir 44 infiltrates the wick 46 through the ends of the wick extending into the reservoir 44 and is drawn along the wick by surface tension / capillary action (i.e. wicking). The aerosol generator 48 in this example comprises an electrically resistive wire coiled around the wick 46. In figure 1, the aerosol generator 48 comprises a nickel chrome alloy (Cr20Ni80) wire and the wick 46 comprises a glass fibre bundle, but the specific aerosol generator configuration is not significant to the principles described. In use, electrical power may be supplied to the aerosol generator 48 to vaporise an amount of aerosol generating material drawn to the vicinity of the aerosol generator 48 by the wick 46. Vaporised aerosol generating material may then become entrained in air drawn along the cartridge airflow path from the vaporisation region towards the mouthpiece outlet 50 for user inhalation.

As noted above, the rate at which aerosol generating material is vaporised by the aerosol generator 48 will depend on the amount (level) of power supplied to the aerosol generator 48. Thus, electrical power can be applied to the aerosol generator 48 to selectively generate aerosol from the aerosol generating material in the cartridge part 4, and furthermore, the rate of aerosol generation can be changed by changing the amount of power supplied to the aerosol generator 48, for example through pulse width and/or frequency modulation techniques.

The reusable part 2 comprises an outer housing 12 having with an opening that defines an air inlet 28 for the e-cigarette, a power source or supply 26 (e.g. a battery) for providing operating power for the electronic cigarette, control circuitry / controller 22 for controlling and monitoring the operation of the electronic cigarette, a first user input button 14, a second user input button 16, and a visual display 24. The outer housing 12 may be formed, e.g. from a plastics or metallic material and in this example has a circular cross section generally conforming to the shape and size of the cartridge part 4, to provide a smooth transition between the two parts 2, 4 at the interface 6. In this example, the reusable part 2 has a length of around 8 cm so the overall length of the e-cigarette when the cartridge part 4 and the reusable part 2 are coupled together is around 12 cm.

As noted above, the power source / supply 26 may be retained within the system 1 by an element such as a housing 12, which may prevent direct access to the power supply 26 (which might be hazardous) by the user during normal use, to minimise the risk of access for potential misuse or tampering. Particularly for all-in-one systems as opposed to two-part systems, the system might be disposed of as a single unit. However, at the end of life of the system, e.g. when the reservoir of aerosol generating material is depleted for an all-in-one disposable system, or when the power supply 26 has reached its lifecycle limit, particularly for a rechargeable power supply 26 that can be used with multiple cartridges, it is desirable to remove the power supply 26 so it can be re-used, recycled or disposed of separately.

As shown schematically in figure 1, the system 1 may comprise a retention mechanism 100 for securing the power supply 26 within the system 1, so it cannot readily/directly be accessed in normal use. The retention mechanism 100 is described in further detail later with reference to the subsequent figures below.

The air inlet 28 connects to an airflow path 51 through the reusable part 2. The reusable part airflow path 51 in turn connects to the cartridge airflow path 52 across the interface 6 when the reusable part 2 and cartridge part 4 are connected together. Thus, when a user inhales on the mouthpiece opening 50, air is drawn in through the air inlet 28, along the reusable part airflow path 51, across the interface 6, through the aerosol generation area in the vicinity of the aerosol generator 48 (where vaporised aerosol generating material becomes entrained in the air flow), along the cartridge airflow path 52, and out through the mouthpiece opening 50 for user inhalation.

The power source 26 in this example is rechargeable and may be a conventional type, e.g. of the kind normally used in electronic cigarettes and other applications requiring provision of relatively high currents over relatively short periods. The power source 26 may be recharged through a charging connector in the reusable part housing 12, for example a USB connector.

Optionally, first and/or second user input buttons 14, 16 may be provided, which in this example are conventional mechanical buttons, e.g. comprising a spring mounted component which may be pressed by a user to establish an electrical contact. The input buttons may be input devices for detecting user input and the manner in which the buttons are implemented is not significant. The buttons may be assigned functions such as switching the system 1 on and off, and/or adjusting user settings such as a power to be supplied from the power source 26 to the aerosol generator 48.

A display 24 may be provided to give a user a visual indication of various characteristics associated with the aerosol delivery system, e.g. current power setting information, remaining power source power, etc. The display may be implemented in various ways. In this example, the display 24 comprises a conventional pixilated LCD screen. In other implementations, the display may comprise one or more discrete indicators, e.g. LEDs, arranged to display information, e.g. through particular colours and/or flash sequences. More generally, the manner in which the display 24 is provided and information is displayed is not significant to the principles described herein - other embodiments may not include a visual display and/or may include other means for providing a user with information relating to operating characteristics of the system 1, e.g. using audio signalling.

A controller 22 is suitably configured / programmed to control the aerosol delivery system 1 to provide functionality as described herein, as well as for providing conventional operating functions of the system 1. The controller (processor circuitry) 22 may be considered to logically comprise various subunits / circuitry elements associated with different aspects of the operation of the system 1. In this example, the controller 22 comprises power supply control circuitry for controlling the supply of power from the power source 26 to the aerosol generator 48 in response to user input, user programming circuitry 20 for establishing configuration settings (e.g. user-defined power settings) in response to user input, as well as other functional units / circuitry associated functionality in accordance with the principles described herein and conventional operating aspects, such as display driving circuitry and user input detection circuitry. The functionality of the controller 22 can be provided in various different ways, e.g. using one or more programmed programmable computer(s) and / or one or more suitably configured application-specific integrated circuit(s) / circuitry / chip(s) / chipset(s). The controller 22 may comprise an application specific integrated circuit (ASIC), CPU, microprocessor or microcontroller. The operations of a controller and other electronic components are generally controlled by software/instructions running on the controller, which may be stored in non-volatile memory, (e.g. ROM), which may be integrated into the controller, or provided separately. The controller 22 may access the ROM to load and execute individual software as and when required.

The reusable part 2 comprises an airflow sensor 30, which is electrically connected to the controller 22. In most embodiments, the airflow sensor 30 comprises a so-called "puff sensor", in that the airflow sensor 30 is used to detect when a user is puffing on the device. In some embodiments, the airflow sensor 30 comprises a switch in an electrical path providing electrical power from the power source 26 to the aerosol generator 48. In such embodiments, the airflow sensor 30 generally comprises a pressure sensor configured to close the switch when subjected to a particular range of pressures, enabling current to flow from the power source 26 to the aerosol generator 48 once the pressure in the vicinity of the airflow sensor 30 drops below a threshold value. The threshold value can be set to a value determined by experimentation to correspond to a characteristic value associated with the initiation of a user puff. In other embodiments, the airflow sensor 30 is connected to the controller 22, and the controller 22 distributes electrical power from the power source 26 to the aerosol generator 48 in dependence of a signal received from the airflow sensor 30 by the controller 22. The specific manner in which the signal output from the airflow sensor 30 (which may comprise a measure of capacitance, resistance or other characteristic of the airflow sensor, made by the controller 22) is used by the controller 22 to control the supply of power from the power source 26 to the aerosol generator 48 can be carried out in accordance with any approach known to the skilled person.

In the example shown in figure 1, the airflow sensor 30 is mounted to an optional printed circuit board (PCB) 31. The airflow sensor 30 may comprise any sensor configured to determine a characteristic of airflow in an airflow path 51 disposed between air inlet 28 and mouthpiece opening 50, e.g. a pressure sensor or transducer (such as a membrane or solid-state pressure sensor), a combined temperature and pressure sensor, or a microphone (e.g. an electret-type microphone), which is sensitive to changes in air pressure, including acoustical signals. The airflow sensor 30 is situated within a sensor cavity or chamber 32, which comprises the interior space defined by one or more chamber walls 34. The sensor cavity 32 comprises a region internal to one or more chamber walls 34 in which an airflow sensor 30 can be fully or partially situated. In some embodiments, the PCB 31 comprises one of the chamber walls of a sensor housing comprising the sensor chamber / cavity 32. A deformable membrane may be disposed across an opening communicating between the sensor cavity 32 containing the sensor 30, and a portion of the airflow path disposed between air inlet 28 and mouthpiece opening 50. The deformable membrane covers the opening, and is attached to one or more of the chamber walls according to approaches described further herein.

The aerosol delivery system 1 may comprise communication circuitry configured to connect to one or more further electronic devices (e.g., a storage / charging case, or a refill / charging dock) to enable data transfer between the system 1 and further electronic device(s). The communication circuitry may be integrated into the controller 22, or implemented separately. The communication circuitry may be configured to support wired or wireless communications between the aerosol delivery system 1 and other electronic devices such as a case, a dock, a computing device such as a smartphone or PC, a base station supporting cellular communications, a relay node providing an onward connection to a base station, a wearable device, or any other portable or fixed device. The controller 22, other components within the system 1 and other devices/systems may comprise one or more processors and data processing may be performed on any of these processors or on a remote processor, the data communicated by wire or wirelessly.

Wireless communications between the aerosol delivery system 1 and a further electronic device may be configured according to data transfer protocols such as Bluetooths, ZigBee, WiFi^{®}, Wifi Direct, GSM, 2G, 3G, 4G, 5G, LTE, NFC, RFID, or generally any other wireless, and/or wired, network protocol or interface. The communication circuitry may comprise any suitable interface for wired data connection, such as USB-C, micro-USB or Thunderbolt interfaces, and may comprise pin or contact pad arrangements configured to engage cooperating pins or contact pads on a dock, case, cable, or other external device which can be connected to the aerosol delivery system 1.

### Retention mechanism

As outlined above, the claimed invention is directed to a retention mechanism 100 for securing a power supply 26 within an aerosol delivery system 1, the retention mechanism 100 comprising first and second components 110, 120 that are removably engagable with one another. The retention mechanism 100 is configured to secure the power supply 26 within the system 1 when the first and second components 110, 120 are engaged. The engagement functionality may be provided e.g. by one or more protrusions configured to engage or interlock with one or more complementary recesses or protrusions.

The retention mechanism 100 is configured to plastically deform or fracture during disengagement. Plastic deformation or fracture generally provides an irreversible change which may occur to either or both of the first and second components 110, 120, and will typically and optionally (although not essentially) substantially prevent or at least make unreliable subsequent re-engagement of the first and second components 110, 120 after disengagement. Optionally, in some embodiments, it might nevertheless be possible to disengage the mechanism 100 using e.g. a bespoke tool or technique, such as a particular sequence of steps that would not be apparent immediately to an end user.

Accordingly, the retention mechanism 100 may in some examples be a single-use retention mechanism 100 that can be engaged once, but is configured to plastically deform or fracture during disengagement, to prevent re-engagement. This can be achieved e.g. by providing a retention mechanism 100 using interlocking components, such as complementary protrusions and recesses on the first and second components 110, 120, and preventing full access to the interlocking components 110, 120 when engaged, so that if the components 110, 120 are moved relative to one another, then one or more of the components 110, 120 will plastically deform or fracture.

Plastic deformation or fracture may be promoted by providing a stress concentration mechanism in the system 1, e.g. on or around any sub-component, part or portion of the retention mechanism 100. Any suitable stress concentrator may be used, such as providing a weakened region/portion 112 e.g. in the form of a different (e.g. brittle) material (such as polycarbonate plastic), a thinner or hollow region, or an undercut or holey region where material is removed or absent, e.g. on a protrusion or recess, or proximal to or surrounding the connecting portion or base of a protrusion or recess, to promote plastic deformation or fracture of/at that protrusion or recess. For a thinner or hollow region, the region may be thinner in any direction, such as width or thickness. A hollow region may e.g. be weakened in contrast to a thicker but still hollow portion or weakened in contrast to a solid (non-hollow) portion. A holey region comprises one, or particularly multiple, holes/apertures. In some examples, the first and/or second components 110, 120 comprise(s) a weakened region 112. Optionally, multiple weakened regions 112 may be provided, e.g. one region for weakening each protrusion or recess.

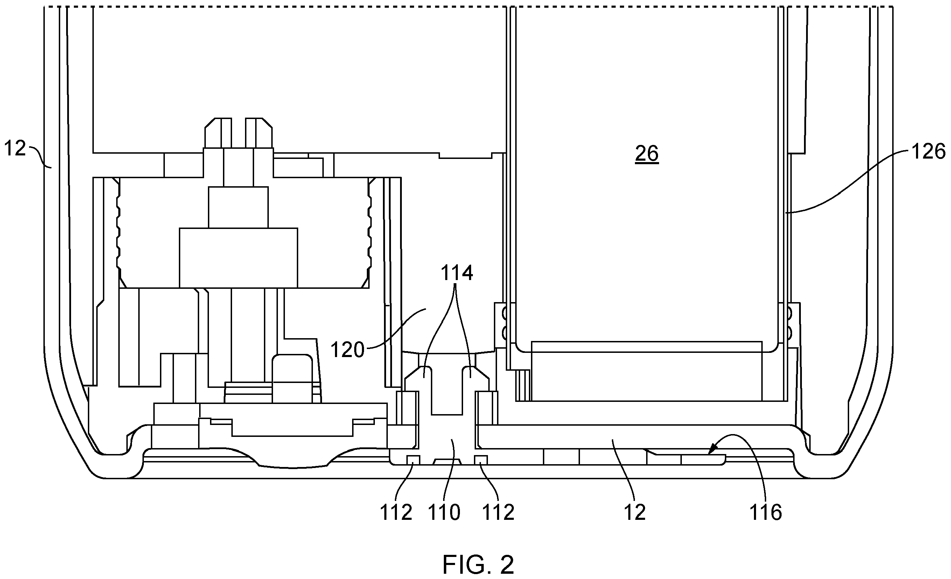

Figure 2 is a schematic cross-section view of an aerosol delivery system 1 comprising a retention mechanism 100 in accordance with some embodiments of the disclosure. In the example of figure 2, the aerosol delivery system 1 comprises a power supply 26 and a retention mechanism 100 comprising first and second components 110, 120 configured to secure the power supply 26 within the system 1 during normal use. The first and second components 110, 120 may take any suitable form, where particular examples are described later with reference to the subsequent figures. Figure 2 illustrates that the system 1 comprises a cavity 126 for receiving the power supply 26, and an outer housing 12 or shell enclosing the power supply 26 within the cavity 126. In this example, the cavity 126 is part of the second component 120. The first component 110 extends through the housing 12 and secures to the second component 120, thereby preventing removal of the housing 12 which encloses the power supply 26.

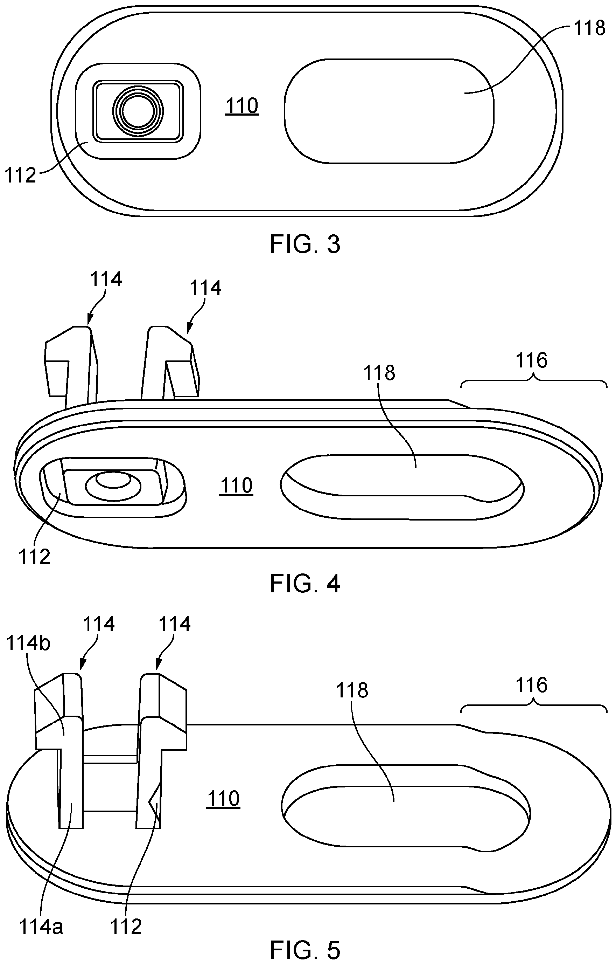

Figures 3-8 illustrate the individual aspects of this example in more detail. Figures 3-5 are top-down, side perspective and bottom-up perspective views respectively of the first component 110, illustrating some optional features of the first component 110. The first component 110 may take any suitable form. In this example, the first component 110 is generally oval in shape, forming a tab and comprises an opposing pair of substantially 'L'-shaped protrusions or lugs 114 proximal to a first end of the tab, extending in the depth direction, perpendicular to the planar oval shape. The protrusions 114 each connect to the tab at a base portion 114a and extend away from the tab to a tip in the form of a foot 114b, forming the 'L'-shape.

In this example, best shown in figures 3-4, the first component 110 comprises a weakened region 112 surrounding the base portions 114a of the protrusions 114, i.e. surrounding a portion of the tab where the L-shaped protrusions 114 project out. Here, the weakened region 112 is in the form of a substantially 50% thinner region relative to the main portion of the tab, forming a groove or channel around the bases 114a of the protrusions 114. Generally, the thinner region is at least 25% thinner, optionally at least 30%, 40%, 50%, 60%, 70% or 80% thinner than the typical thickness of the first component 110 (e.g. the thickness away from the protrusions 114). The weakened region 112 is configured to provide a stress concentration, to promote plastic deformation or fracture of the retention mechanism 100 during attempted disengagement. In some examples, the weakened region 112 comprises an undercut. Figure 5 illustrates an optional triangular undercut on the right-hand protrusion 114 of the first component 110, weakening the base 114a of this protrusion 114 and making it particularly prone to fracture.

The first component 110 also comprises other optional features to aid disengagement of the retention mechanism 100 by the user. As shown in figures 3-5, the first component 110 may comprise an aperture 118. The aperture 118 provides an opening for receiving the user's fingertip/fingernail to aid them in moving the first component 110 relative to the second component 120. In this example, the user may pull the first component 110 away from the system 1 or rotate the first component 110 with respect to the second component 120 to disengage the mechanism 100. The aperture 118 thus provides ergonomic benefits, enhancing ease of use.

As best shown in figures 4-5, the first component 110 may comprise a stepped portion 116, here shown proximal to a second end, away from the first end that is proximal to the protrusions 114. As shown in figure 2, the stepped portion 116 spaces the second end of the tab (distal from the protrusions 114) from the housing 12, aiding a user in leveraging the second end to pull the first component 110 away from the rest of the system 1 (here, more specifically, away from the housing 12, which encloses the power supply 26 within the system 1, and away from the second component 120). Accordingly, in this example, the first component 110 forms a pull-tab, where when pulling the tab away from the system 1, the tab acts as a lever and is designed to deform or fracture the weakest part of the mechanism 100, which can be designed suitably. The weakest part may generally be one or more protrusions or recesses on the first or second components 110, 120. In particular, as illustrated in this example, the first component 110 may be designed so that the weakest part is a protrusion 114 on the first component 110, so that the first component 110 is fractured or at least plastically deformed upon movement relative to the second component 120. Nevertheless, it should be readily understood that the second component 120 may instead be designed to be plastically deformed or fractured.

In some examples, the plastic deformation or fracture may be designed to occur only following one or more particular movement trajectories or in one or more degrees of freedom, such as linear, torsional or rotational movement, or any combination thereof, and optionally not to occur following one or more (other) particular movement trajectories or in one or more degrees of freedom.

In some examples, when the first and second components 110, 120 are engaged, the retention mechanism 100 is configured to plastically deform or fracture upon:
- a substantially linear movement of the first component 110 relative to the second component 120 (e.g. upon axial separation along or perpendicular to an axis of engagement); and/or
- a substantially torsional movement of the first component 110 relative to the second component 120; and/or
- a substantially rotational movement of the first component 110 relative to the second component 120.

In some examples, when the first and second components 110, 120 are engaged, the retention mechanism 100 is configured not to plastically deform or fracture upon a substantially linear, torsional or rotational movement of the first component 110 relative to the second component 120, where the latter may be implemented e.g. by providing rounded protrusions / recesses that may rotate freely with respect to one another without disengaging.

Any suitable combinations of the above may be utilised, which may beneficially allow relative movement along one or more particular trajectories or in one or more degrees of freedom, reducing the risk of inadvertently disengaging the mechanism 100 and causing plastic deformation/fracture, whilst still providing the required functionality of plastically deforming or fracturing during intentional attempted disengagement. In particular, the system 1 may be designed not to plastically deform or fracture upon a substantially rotational movement of the first component 110 relative to the second component 120, but to plastically deform or fracture upon a substantially linear movement of the first component 110 relative to the second component 120.

Although not illustrated, the first component 110 may form a ring-pull (e.g. as used to open a beverage can), which fractures (pierces) another component, which here may be the relevant sub-component of the system 1 enclosing the power supply 26 (e.g. a housing), to allow removal of the power supply 26. The ring-pull first component 110 may also be designed to fracture itself and/or detach from the system 1, to prevent re-use.

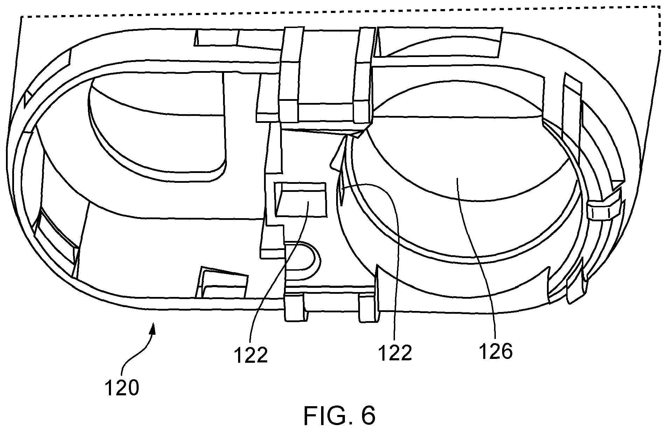

Figure 6 is a perspective view of a second component 120 in accordance with some embodiments of the disclosure, illustrating some optional features of the second component 120.

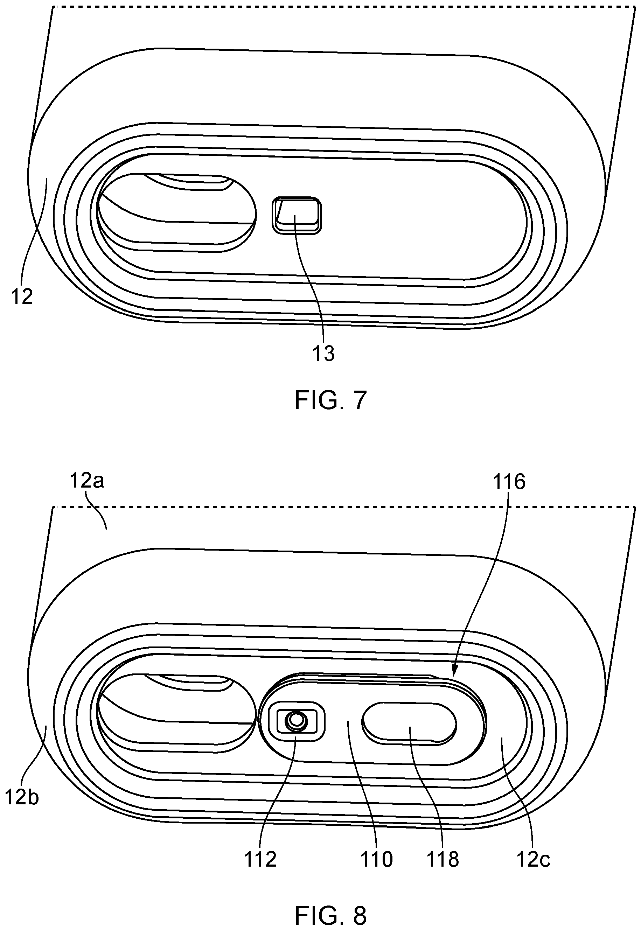

In this example, the second component comprises a retaining body 120 (or inner bracket/housing) having a power supply cavity 126, for receiving the power supply 26. Here, the power supply 26 is an elongate cylinder and thus the cavity 126 is correspondingly cylindrically shaped to receive the cylindrical power supply 26. In this example, the power supply 26 is not directly secured within the cavity 126 by the second component 120, rather it is secured by the outer housing 12, as shown in figure 2 and described later with reference to figures 7-8. Nevertheless, in further embodiments, the power supply 26 may be secured within the cavity 126 by the second component 120 or by another sub-component, e.g. to aid assembly.

In this example, the second retaining body component 120 comprises a connection portion for engaging the first component 110, the connection portion comprising a first recess 122 extending axially into the body 120, and a pair of second opposing side recesses 122 (only one is shown in figure 6), which receive the feet 114b of the L-shaped protrusions 114 of the first component 110 in use, to provide a secure snap-fit engagement. In other examples, the second component 120 may comprise one or more complementary protrusions configured to engage with one or more recesses or protrusions of the first component 110.

Figure 7 is a perspective view of a housing 12 in accordance with some embodiments of the disclosure and figure 8 is a perspective view of a first component 110 engaging the second component 120 through the housing 12 of figure 7. As shown in figures 7-8, the system 1 may comprise a housing 12 which forms a shell around the second component 120, here enclosing the power supply 26 in the system 1 and preventing direct access thereto during normal use. The housing 12 may comprise a single part 12 or multiple parts, such as a tubular side part 12a and one or more end caps 12b, as depicted in figure 8. As also shown in figures 7-8, the housing 12 may comprise one or more apertures 13 to allow the retention mechanism 100 to engage through the housing 12. As shown in the engaged configuration of figure 8, the protrusions 114 of the first component 110 pass through the housing aperture 13 and are received by the second component recesses 122 (shown in figure 6), to provide a secure snap-fit engagement between the first and second components 110, 120, securing the housing 12, which prevents direct access for the user to the power supply 26. In other words, the first component 110 secures to the second component 120 via the housing 12, thereby preventing removal of the housing 12 which encloses the power supply 26.

As shown in figures 7-8 and earlier figure 2, the housing 12 may provide a cavity or recess 12c for accommodating the first component 110, so that the first component 110 does not project beyond the housing 12, thus is less exposed and less prone to being moved or dislodged accidentally, reducing the risk of inadvertent partial or full disengagement of the retention mechanism 100.

Although not shown, in some embodiments, the system 1 may further comprise a cover over/enclosing the first component 110, similarly reducing the risk of inadvertent partial or full disengagement of the retention mechanism 100. The cover may be secured in place by another engagement mechanism (e.g. comprising protrusions and/or recesses) or a fastener. The fastener may be single-use such as a rivet or staple, or reusable, such as a turnbuckle, bolt or screw.

Following the above, it should be understood that a power supply 26 can be secured within an aerosol delivery system 1 using a retention mechanism 100, the retention mechanism 100 comprising a first component 110 (such as a retention element) and a second component 120 (such as a retaining body, which may have a connection portion) that is removably engagable with the first component 110. The securement method involves locating the power supply 26 in the system 1 (e.g. in a cavity 126 of the second component 120) and engaging the first component 110 with the second component 120 to secure the power supply 26 within the system 1, preventing direct user access to the power supply 26 in normal use. The retention mechanism 100 is configured to plastically deform or fracture during disengagement, which will typically prevent re-engagement of the first component 110 with the second component 120 after disengagement, but this is not strictly essential.

Following the above, it should also be understood that from the assembled state (best shown in figure 2), the power supply 26 can be removed by disengaging the first and second components 110, 120. As noted above, in the illustrated example, the first component 110 is a pull-tab having a stepped portion 116 spacing the second, opposing end of the tab from the housing 12. Accordingly, in this example, the user can lever the pull-tab first component 110 away from the system 1, which will typically deform or fracture the weakest part of the mechanism 100. In the illustrated example, the L-shaped protrusions 114 on the first component 110 are weakened by the thinner portion 112 surrounding the bases 114a of the protrusions 114, which thus readily plastically deform or fracture when the first component 110 is levered away from the system 1. This allows the first component 110 to be removed, so that the housing 12 can be removed (as it is no longer secured by the first component 110) and the power supply 26 can then be extracted from the cavity 126 for recycling.

Although not shown in the depicted example, the first component 110 may itself enclose the power supply 26 within the system 1 to prevent direct access to the power supply 26 by the user in normal use. For example, the first component 110 may comprise a housing (or other portion or element) for enclosing the power supply 26 within the system 1. As an example, referring to figure 8, the first component 110 may comprise an end cap 12b enclosing the power supply 26. In any case, the first component 110 remains removably engagable with the second component 120, and either the first component 110 or the second component 120 is configured to plastically deform or fracture during disengagement, allowing removal of the part of the system 1 enclosing the power supply 26, so that the power supply 26 can be accessed and removed for recycling when the system 1 or the power supply 26 reaches end-of-life.

In some embodiments, the system 1 comprises a controller 22 configured to detect disengagement of the retention mechanism 100, and in response disable the supply of power from the power supply 26 to the system 1, e.g. preventing power supply to the aerosol generator 48. Detection by the controller 22 may be implemented e.g. using one or more sensors configured to detect engagement and/or disengagement, or by providing an electrical pathway through the retention mechanism 100 that is broken upon disengagement, thus is detectable by the controller 22.

The various embodiments described herein are presented only to assist in understanding and teaching the claimed features. These embodiments are provided as a representative sample of embodiments only, and are not exhaustive and/or exclusive. Any functions of a processor (e.g. controller) may be shared between processors on the various devices/systems in the wider system and/or a remote server. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention.

Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future. Protection may also be sought for any features disclosed in any one or more published documents referenced herein in combination with the present disclosure.

### Terminology

### Delivery System

As used herein, the term "delivery system" is intended to encompass systems that deliver at least one substance to a user in use, and includes:
combustible aerosol provision systems, such as cigarettes, cigarillos, cigars, and tobacco for pipes or for roll-your-own or for make-your-own cigarettes (whether based on tobacco, tobacco derivatives, expanded tobacco, reconstituted tobacco, tobacco substitutes or other smokable material);
non-combustible aerosol provision systems that release compounds from an aerosol-generating material without combusting the aerosol-generating material, such as electronic cigarettes, tobacco heating products, and hybrid systems to generate aerosol using a combination of aerosol-generating materials; and
aerosol-free delivery systems that deliver the at least one substance to a user orally, nasally, transdermally or in another way without forming an aerosol, including but not limited to, lozenges, gums, patches, articles comprising inhalable powders, and oral products such as oral tobacco which includes snus or moist snuff, wherein the at least one substance may or may not comprise nicotine.

### Combustible Aerosol Provision System

According to the present disclosure, a "combustible" aerosol provision system is one where a constituent aerosol-generating material of the aerosol provision system (or component thereof) is combusted or burned during use in order to facilitate delivery of at least one substance to a user.

In some embodiments, the delivery system is a combustible aerosol provision system, such as a system selected from the group consisting of a cigarette, a cigarillo and a cigar. In some embodiments, the disclosure relates to a component for use in a combustible aerosol provision system, such as a filter, a filter rod, a filter segment, a tobacco rod, a spill, an aerosol-modifying agent release component such as a capsule, a thread, or a bead, or a paper such as a plug wrap, a tipping paper or a cigarette paper.

### Non-Combustible Aerosol Provision System

According to the present disclosure, a "non-combustible" aerosol provision system is one where a constituent aerosol-generating material of the aerosol provision system (or component thereof) is not combusted or burned in order to facilitate delivery of at least one substance to a user.

In some embodiments, the delivery system is a non-combustible aerosol provision system, such as a powered non-combustible aerosol provision system. In some embodiments, the non-combustible aerosol provision system is an electronic cigarette, also known as a vaping device or electronic nicotine delivery system (END), although it is noted that the presence of nicotine in the aerosol-generating material is not a requirement. In some embodiments, the non-combustible aerosol provision system is an aerosol-generating material heating system, also known as a heat-not-burn system. An example of such a system is a tobacco heating system.

In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material. The solid aerosol-generating material may comprise, for example, tobacco or a non-tobacco product.

Typically, the non-combustible aerosol provision system may comprise a non-combustible aerosol provision device and a consumable for use with the non-combustible aerosol provision device. In some embodiments, the disclosure relates to consumables comprising aerosol-generating material and configured to be used with non-combustible aerosol provision devices. These consumables are sometimes referred to as articles throughout the disclosure.

In some embodiments, the non-combustible aerosol provision system, such as a non-combustible aerosol provision device thereof, may comprise a power source and a controller. The power source may, for example, be an electric power source or an exothermic power source. In some embodiments, the exothermic power source comprises a carbon substrate which may be energised so as to distribute power in the form of heat to an aerosol-generating material or to a heat transfer material in proximity to the exothermic power source.

In some embodiments, the non-combustible aerosol provision system may comprise an area for receiving the consumable, an aerosol generator, an aerosol generation area, a housing, a mouthpiece, a filter and/or an aerosol-modifying agent. In some embodiments, the consumable for use with the non-combustible aerosol provision device may comprise aerosol-generating material, an aerosol-generating material storage area, an aerosol-generating material transfer component, an aerosol generator, an aerosol generation area, a housing, a wrapper, a filter, a mouthpiece, and/or an aerosol-modifying agent.

### Aerosol-Free Delivery System

In some embodiments, the delivery system is an aerosol-free delivery system that delivers at least one substance to a user orally, nasally, transdermally or in another way without forming an aerosol, including but not limited to, lozenges, gums, patches, articles comprising inhalable powders, and oral products such as oral tobacco which includes snus or moist snuff, wherein the at least one substance may or may not comprise nicotine.

In some embodiments, the substance to be delivered may be an aerosol-generating material or a material that is not intended to be aerosolised. As appropriate, either material may comprise one or more active constituents, one or more flavours, one or more aerosol-former materials, and/or one or more other functional materials.

### Active Substance

In some embodiments, the substance to be delivered comprises an active substance. The active substance as used herein may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical. In one embodiment the active substance is a legally permissible recreational drug. In some embodiments, the active substance comprises nicotine. In some embodiments, the active substance comprises caffeine, melatonin or vitamin B12. As noted herein, the active substance may comprise one or more constituents, derivatives or extracts of cannabis, such as one or more cannabinoids or terpenes. The active substance may be CBD or a derivative thereof. As noted herein, the active substance may comprise or be derived from one or more botanicals or constituents, derivatives or extracts thereof. As used herein, the term "botanical" includes any material derived from plants including, but not limited to, extracts, leaves, bark, fibres, stems, roots, seeds, flowers, fruits, pollen, husk, shells or the like. Alternatively, the material may comprise an active compound naturally existing in a botanical, obtained synthetically. The material may be in the form of liquid, gas, solid, powder, dust, crushed particles, granules, pellets, shreds, strips, sheets, or the like.

Example botanicals are tobacco, eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens.

In some embodiments, the active substance comprises or is derived from one or more botanicals or constituents, derivatives or extracts thereof and the botanical is tobacco. In some embodiments, the active substance comprises or derived from one or more botanicals or constituents, derivatives or extracts thereof and the botanical is selected from eucalyptus, star anise, cocoa and hemp. In some embodiments, the active substance comprises or derived from one or more botanicals or constituents, derivatives or extracts thereof and the botanical is selected from rooibos and fennel.

### Flavours

In some embodiments, the substance to be delivered comprises a flavour. As used herein, the terms "flavour" and "flavourant" refer to materials which, where local regulations permit, may be used to create a desired taste, aroma or other somatosensorial sensation in a product for adult consumers. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In some embodiments, the flavour comprises menthol, spearmint and/or peppermint. In some embodiments, the flavour comprises flavour components of cucumber, blueberry, citrus fruits and/or redberry. In some embodiments, the flavour comprises eugenol. In some embodiments, the flavour comprises flavour components extracted from tobacco. In some embodiments, the flavour comprises flavour components extracted from cannabis.

In some embodiments, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucolyptol, WS-3.

### Aerosol-generating material

Aerosol-generating material is a material that is capable of generating aerosol, for example when heated, irradiated or energized in any other way. Aerosol-generating material may, for example, be in the form of a solid, liquid or semi-solid (such as a gel) which may or may not contain an active substance and/or flavourants. The aerosol-generating material may comprise one or more active substances and/or flavours, one or more aerosol-former materials, and optionally one or more other functional material.

The aerosol-generating material may comprise a binder, such as a gelling agent, and an aerosol former. Optionally, a substance to be delivered and/or filler may also be present. Optionally, a solvent, such as water, is also present and one or more other components of the aerosol-generating material may or may not be soluble in the solvent. In some embodiments, the aerosol-generating material is substantially free from botanical material. In particular, in some embodiments, the aerosol-generating material is substantially tobacco free.

The aerosol-generating material may comprise or be in the form of an aerosol-generating film. The aerosol-generating film may comprise a binder, such as a gelling agent, and an aerosol former. Optionally, a substance to be delivered and/or filler may also be present. The aerosol-generating film may be substantially free from botanical material. In particular, in some embodiments, the aerosol-generating material is substantially tobacco free. The aerosol-generating film may have a thickness of about 0.015 mm to about 1 mm. For example, the thickness may be in the range of about 0.05 mm, 0.1 mm or 0.15 mm to about 0.5 mm or 0.3 mm. The aerosol-generating material may comprise more than one film, and the thickness described herein may refer to the aggregate thickness of those films.

The aerosol-generating film may be continuous. For example, the film may comprise or be a continuous sheet of material. The sheet may be in the form of a wrapper, it may be gathered to form a gathered sheet or it may be shredded to form a shredded sheet. The shredded sheet may comprise one or more strands or strips of aerosol-generating material. The aerosol-generating film may be discontinuous. For example, the aerosol-generating film may comprise one or more discrete portions or regions of aerosol-generating material, such as dots, stripes or lines, which may be supported on a support. In such embodiments, the support may be planar or non-planar.

The aerosol-generating film may be formed by combining a binder, such as a gelling agent, with a solvent, such as water, an aerosol-former and one or more other components, such as one or more substances to be delivered, to form a slurry and then heating the slurry to volatilise at least some of the solvent to form the aerosol-generating film. The slurry may be heated to remove at least about 60 wt%, 70 wt%, 80 wt%, 85 wt% or 90 wt% of the solvent.

The aerosol-generating material may comprise or be an "amorphous solid". In some embodiments, the aerosol-generating materiel comprises an aerosol-generating film that is an amorphous solid. The amorphous solid may be a "monolithic solid". The amorphous solid may be substantially non-fibrous. In some embodiments, the amorphous solid may be a dried gel. The amorphous solid is a solid material that may retain some fluid, such as liquid, within it. In some embodiments, the amorphous solid may, for example, comprise from about 50wt%, 60wt% or 70wt% of amorphous solid, to about 90wt%, 95wt% or 100wt% of amorphous solid.

The amorphous solid may be substantially free from botanical material. The amorphous solid may be substantially tobacco free.

### Aerosol-former material

The aerosol-former material may comprise one or more constituents capable of forming an aerosol.

In some embodiments, the aerosol-former material may comprise one or more of glycerol, propylene glycol, diethylene glycol, triethylene glycol, tetraethylene glycol, 1 ,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

### Functional material

The one or more other functional materials may comprise one or more of pH regulators, colouring agents, preservatives, binders, fillers, stabilizers, and/or antioxidants.

### Substrate

The material may be present on or in a support, to form a substrate. The support may, for example, be or comprise paper, card, paperboard, cardboard, reconstituted material, a plastics material, a ceramic material, a composite material, glass, a metal, or a metal alloy. In some embodiments, the support comprises a susceptor. In some embodiments, the susceptor is embedded within the material. In some alternative embodiments, the susceptor is on one or either side of the material.

### Consumable

A consumable is an article comprising or consisting of aerosol-generating material, part or all of which is intended to be consumed during use by a user. A consumable may comprise one or more other components, such as an aerosol-generating material storage area, an aerosol-generating material transfer component, an aerosol generation area, a housing, a wrapper, a mouthpiece, a filter and/or an aerosol-modifying agent. A consumable may also comprise an aerosol generator, such as a heater, that emits heat to cause the aerosol-generating material to generate aerosol in use. The heater may, for example, comprise combustible material, a material heatable by electrical conduction, or a susceptor.

### Susceptor

A susceptor is a material that is heatable by penetration with a varying magnetic field, such as an alternating magnetic field. The susceptor may be an electrically-conductive material, so that penetration thereof with a varying magnetic field causes induction heating of the heating material. The heating material may be magnetic material, so that penetration thereof with a varying magnetic field causes magnetic hysteresis heating of the heating material. The susceptor may be both electrically-conductive and magnetic, so that the susceptor is heatable by both heating mechanisms. The device that is configured to generate the varying magnetic field is referred to as a magnetic field generator, herein.

### Aerosol-modifying agent

An aerosol-modifying agent is a substance, typically located downstream of the aerosol generation area, that is configured to modify the aerosol generated, for example by changing the taste, flavour, acidity or another characteristic of the aerosol. The aerosol-modifying agent may be provided in an aerosol-modifying agent release component, that is operable to selectively release the aerosol-modifying agent. The aerosol-modifying agent may, for example, be an additive or a sorbent. The aerosol-modifying agent may, for example, comprise one or more of a flavourant, a colourant, water, and a carbon adsorbent. The aerosol-modifying agent may, for example, be a solid, a liquid, or a gel. The aerosol-modifying agent may be in powder, thread or granule form. The aerosol-modifying agent may be free from filtration material.

### Aerosol generator

An aerosol generator is an apparatus configured to cause aerosol to be generated from the aerosol-generating material. In some embodiments, the aerosol generator is a heater configured to subject the aerosol-generating material to heat energy, so as to release one or more volatiles from the aerosol-generating material to form an aerosol. In some embodiments, the aerosol generator is configured to cause an aerosol to be generated from the aerosol-generating material without heating. For example, the aerosol generator may be configured to subject the aerosol-generating material to one or more of vibration, increased pressure, or electrostatic energy.

The present disclosure relates to aerosol delivery systems (which may also be referred to as vapour delivery systems) such as nebulisers or e-cigarettes. Throughout the following description the term "e-cigarette" or "electronic cigarette" may sometimes be used, but it will be appreciated this term may be used interchangeably with aerosol delivery system / device and electronic aerosol delivery system / device. Furthermore, and as is common in the technical field, the terms "aerosol" and "vapour", and related terms such as "vaporise", "volatilise" and "aerosolise", may generally be used interchangeably.

Aerosol delivery systems (e-cigarettes) often, though not always, comprise a modular assembly comprising a reusable device part and a replaceable (disposable/consumable) cartridge part. Often, the replaceable cartridge part will comprise the aerosol generating material and the vaporiser (which may collectively be called a 'cartomizer') and the reusable device part will comprise the power supply (e.g. rechargeable power source) and control circuitry. It will be appreciated these different parts may comprise further elements depending on functionality. For example, the reusable device part will often comprise a user interface for receiving user input and displaying operating status characteristics, and the replaceable cartridge device part in some cases comprises a temperature sensor for helping to control temperature. Cartridges are electrically and mechanically coupled to the control unit for use, for example using a screw thread, bayonet, or magnetic coupling with appropriately arranged electrical contacts. When the aerosol generating material in a cartridge is exhausted, or the user wishes to switch to a different cartridge having a different aerosol generating material, the cartridge may be removed from the reusable part and a replacement cartridge attached in its place. Systems and devices conforming to this type of two-part modular configuration may generally be referred to as two-part systems/devices.

It is common for electronic cigarettes to have a generally elongate shape. For the sake of providing a concrete example, certain embodiments of the disclosure will be taken to comprise this kind of generally elongate two-part system employing disposable cartridges. However, it will be appreciated that the underlying principles described herein may equally be adopted for different configurations, for example single-part systems or modular systems comprising more than two parts, refillable devices and single-use disposables, as well as other overall shapes, for example based on so-called box-mod high performance devices that typically have a boxier shape. More generally, it will be appreciated certain embodiments of the disclosure are based on aerosol delivery systems which are operationally configured to provide functionality in accordance with the principles described herein and the constructional aspects of systems configured to provide the functionality in accordance with certain embodiments of the disclosure is not of primary significance.

Throughout the disclosure, the terms `substantially', 'approximately' and 'about' should be considered to mean within +/- 10% unless indicated otherwise.

### Index to reference numerals

- 1: aerosol delivery system
- 2: reusable part
- 4: cartridge part
- 6: interface between reusable part and cartridge part
- 12: housing
- 13: housing aperture
- 14, 16: user input buttons
- 20: user programming circuitry
- 22: controller
- 24: display
- 26: power source
- 28: air inlet
- 30: airflow sensor
- 31: printed circuit board (PCB)
- 32: sensor cavity or chamber
- 34: chamber wall
- 42: cartridge housing
- 44: chamber or reservoir
- 46: wick
- 48: aerosol generator
- 50: mouthpiece outlet
- 51: airflow path through reusable part
- 52: airflow path through cartridge
- 100: retention mechanism
- 110: first component
- 112: first component weakened region
- 114: first component protrusion
- 116: first component stepped portion
- 118: first component aperture
- 120: second component
- 122: second component recess
- 126: power supply cavity

## Claims

1. An aerosol delivery system comprising:
a power supply; and
a retention mechanism for securing the power supply within the system, wherein the retention mechanism comprises:
a first component and a second component removably engagable with the first component, wherein the retention mechanism is configured to plastically deform or fracture during disengagement.

2. The aerosol delivery system of claim 1, wherein the retention mechanism is a single-use retention mechanism.

3. The aerosol delivery system of any preceding claim, wherein the retention mechanism comprises a weakened region configured to promote the plastic deformation or fracture during disengagement.

4. The aerosol delivery system of claim 3, wherein the weakened region comprises a thinner region, a hollow region, a holey region and/or an undercut.

5. The aerosol delivery system of any preceding claim, wherein the first or second component comprises one or more protrusions, configured to engage one or more complementary recesses or protrusions on the other of the first and second components.

6. The aerosol delivery system of claim 5, wherein the first or second component comprises a thinner region of, proximal to or surrounding the one or more protrusions, providing a stress concentration on, proximal to or surrounding the one or more protrusions.

7. The aerosol delivery system of any preceding claim, wherein the first or second component comprises a cavity for receiving the power supply.

8. The aerosol delivery system of claim 7, wherein the system, the first or the second component comprises a housing enclosing the power supply within the cavity.

9. The aerosol delivery system of any preceding claim, wherein the first component comprises:
a. one or more protrusions or recesses at or proximal to a first end of the first component, the one or more protrusions or recesses configured to engage with the second component; and
b. a stepped portion at or proximal to a second, opposing end of the first component.

10. The aerosol delivery system of claim 9, wherein the stepped portion spaces the second, opposing end of the first component from a housing enclosing the power supply within the cavity.

11. The aerosol delivery system of any preceding claim, wherein the first component comprises a pull-tab or ring-pull.

12. The aerosol delivery system of any preceding claim, wherein:
a. the first or second component comprises one or more protrusions configured to engage the other of the first and second components;
b. the first or second component comprises a cavity for receiving the power supply; and
c. the first or second component comprises a weakened region configured to promote plastic deformation or fracture of the retention mechanism during disengagement of the retention mechanism, preventing subsequent re-engagement of the retention mechanism.

13. The aerosol delivery system of claim 12, wherein:
a. the system comprises a housing enclosing the power supply within the cavity; and
b. the housing comprises an aperture, wherein when the retention mechanism is engaged, the one or more protrusions of the first or second component pass through the aperture in the housing to engage the other of the first and second components.

14. The aerosol delivery system of any preceding claim, further comprising an aerosol generator.

15. A method of securing a power supply within an aerosol delivery system, comprising:
providing a power supply and a retention mechanism comprising a first component and a second component removably engagable with the first component, the method comprising:
locating the power supply within the system; and
engaging the first component with the second component to secure the power supply within the system, wherein the retention mechanism is configured to plastically deform or fracture during disengagement.
